# EUROPÄISCHE PATENTSCHRIFT

(11) **EP 1 531 778 B1**
(45) Veröffentlichungstag und Bekanntmachung des Hinweises auf die Patenterteilung: **02.11.2011**
(21) Anmeldenummer: 03792084.0
(22) Anmeldetag: 15.08.2003
(51) Int. Cl.: A61K 6/08

(54) **PRÄPARAT ZUR BEFESTIGUNG AN EINEM NATÜRLICHEN ZAHNTEIL ODER ZAHN UND ENTSPRECHENDES BEFESTIGUNGSVERFAHREN**
PREPARATION FOR FIXING TO A NATURAL PIECE OF TOOTH OR TO A TOOTH AND CORRESPONDING FIXING METHOD
PREPARATION DESTINEE A ETRE FIXEE SUR UNE PARTIE DE DENT NATURELLE OU SUR UNE DENT

(30) Priorität: 23.08.2002 CH 146002
(43) Veröffentlichungstag der Anmeldung: 25.05.2005
(73) Patentinhaber: Woodwelding AG, 6304 Zug (CH)
(72) Erfinder: MAYER, Jörg, CH-5702 Niederlenz (CH); AESCHLIMANN, Marcel, CH-2514 Ligerz (CH); TORRIANI, Laurent, CH-2516 Lamboing (CH)
(74) Vertreter: Frei Patent Attorneys
(86) Internationale Anmeldenummer: PCT/CH2003/000549
(87) Internationale Veröffentlichungsnummer: WO 2004/017927

(56) Entgegenhaltungen:
- GB-A- 2 277 448
- US-A- 3 919 775
- US-A- 4 407 793
- US-A1- 2001 000 486

## Beschreibung

Die Erfindung liegt im Bereiche der Zahnmedizin und betrifft ein Präparat nach dem Oberbegriff des ersten, unabhängigen Patentanspruchs. Das erfindungsgemässe Präparat eignet sich zur Befestigung an einem natürlichen Zahnteil oder Zahn, es eignet sich insbesondere, als künstlicher Ersatz eines lasttragenden Zahnteils. Die Erfindung betrifft ferner ein Verfahren nach dem Oberbegriff des entsprechenden, unabhängigen Patentanspruchs, welches Verfahren dem Befestigen des Präparates an einem natürlichen Zahnteil oder Zahn, insbesondere dem Ersetzen eines lasttragenden Zahnteils (z.B. endodontische Restauration) dient, wobei das erfindungsgemässe Präparat an einem noch vorhandenen, natürlichen Zahnteil befestigt, beziehungsweise in diesem verankert wird.

Zahnreparaturen, die von einem noch mindestens teilweise vorhandenen Zahn, das heisst von einem natürlichen Zahnteil ausgehen, in denen also eine natürlicher Zahnteil durch einen künstlichen Zahnteil ersetzt wird, betreffen beispielsweise das Füllen ausgebohrter Zähne, das Einsetzen von Inlays, das Aufsetzen von Kronen, Abutments (Unterbauelement für Krone), Brücken, Teilprothesen oder Konstruktionselementen (Unterbauelemente für Prothesen) auf natürlichen Zahnstümpfen oder Zahnwurzeln, das Setzen von Wurzelstiften oder ähnlichen Befestigungsmitteln, die in natürlichen Zahnwurzeln oder Teilen davon eingesetzt werden und der Befestigung von beispielsweise künstlichen Zähnen, Brücken, Konstruktionselementen oder Zahnprothesen dienen. Andererseits ist es auch bekannt, an ganzen, natürlichen Zähnen beispielsweise Verblendungen, Schmuckelemente oder Fixierelemente für Drähte, die einer Zahnkorrektur dienen, zu befestigen.

Für das Füllen ausgebohrter, natürlicher Zähne, für das Aufkronen natürlicher Zahnstümpfe und für das Befestigen eines Wurzelstiftes auf einer natürlichen Zahnwurzel werden gemäss dem Stande der Technik Teile aus Metallen, Polymeren, keramischen Materialien oder Verbundwerkstoffen (Füllungskörper, Krone, Wurzelstift) mit Hilfe von polymeren Zementen am oder im natürlichen Zahnteil befestigt. Dabei werden die Zemente in einem viskosen Zustand eingesetzt und in situ beispielsweise mit ultraviolettem Licht ausgehärtet. Nach der vollständigen Aushärtung können diese Zemente, wie der eingesetzte Teil den Anforderungen der hohen Belastung, denen Zähne ausgesetzt sind, genügen.

Die Aushärtung der Zemente ist aber häufig mit einer Schrumpfung verbunden. Durch diese Schrumpfung entstehen zwischen natürlichem und künstlichem Zahnteil Risse, in die Feuchtigkeit und Bakterien eindringen. Durch die Feuchtigkeit kann der Zement quellen, wodurch der Zahn irreversibel beschädigt werden kann. Die Bakterien verursachen Karies am natürlichen Zahnteil. Zudem sind die ausgehärteten Zemente üblicherweise sehr spröde und Spannungen, die durch Schrumpfung und/oder Quellung entstehen, können nicht oder eben nur durch Rissbildung abgebaut werden. Die genannten Phänomene bewirken, dass beispielsweise die mit polymeren Zementen befestigten Zahnfüllungen gegenüber Amalgamfüllungen zwar weniger giftig sind aber eine weniger hohe Lebensdauer haben.

Um die Zementschrumpfung so gering wie möglich zu machen, werden die Zemente auch bereits teilweise vernetzt eingesetzt. Dieser Lösung des Schrumpfungsproblems sind aber enge Grenzen gesetzt, denn je mehr vernetzt der Zement eingesetzt wird, desto viskoser ist er und desto schwieriger wird es, mit diesem Zement sicher zu stellen, dass er die zu füllenden Hohlräume vollständig ausfüllt.

In der Publikation US-5244933 wird vorgeschlagen, polymere Zemente für den Dentalbereich, die zur Verbesserung der in gehärtetem Zustand erreichbaren, mechanischen Eigenschaften einen hohen Anteil an anorganischen Partikeln enthalten und dadurch ebenfalls sehr hoch viskos sind, in situ mittels hochfrequenter Vibrationen in einen besser fliessenden Zustand zu bringen. Der Effekt beruht auf den thixotropen Eigenschaften derartiger Zemente und die Verflüssigung ist nicht mit einer Wärmeentwicklung verbunden. Das Problem der Schrumpfung wird dadurch aber in keiner Weise gelöst.

Schrumpfung kann nicht toleriert werden, beispielsweise wenn es gilt, den Wurzelkanal abzudichten. Aus diesem Grunde werden für diese Aufgabe auch nicht die oben genannten Zemente benutzt sondern beispielsweise Guttapercha oder andere thermoplastische Polymere mit ähnlichen Eigenschaften wie Guttapercha. Das Polymer wird für die Anwendung erwärmt und dadurch in einen plastischen Zustand gebracht und lediglich durch Abkühlen wieder verfestigt. Die Schrumpfung ist in einem solchen Falle bedeutend kleiner als beim Aushärten durch Vernetzung. Derartige Verfahren zur Abdichtung von Wurzelkanälen sind beispielsweise beschrieben in den Publikationen US-3919775 und US-4525147. Gemäss diesen Publikationen wird ein Guttapercha-Stopfen in den Wurzelkanal eingebracht und als Ganzes mit Wärme und/oder Ultraschall in einen plastischen Zustand gebracht und in den Kanal gepresst. Dies ist ohne unzumutbare thermische Belastung aber nur möglich, wenn das Material eine niedrige Erweichungstemperatur hat (Guttapercha: 70 bis 100°C). Die Erweichungstemperatur beschränkt also die Materialauswahl. Die Funktion der Guttaperchastopfen ist die Abdichtung des Wurzelkanals. Der Stopfen ist in keiner Weise mechanisch belastet. Er wäre dazu wegen der beschränkten mechanischen Festigkeit des verwendeten Materials auch nicht im Stande, auch wenn er, wie in US-4525147 vorgeschlagen, Kohlenfasem enthält.

Auch in der Publikation US-5803736 wird eine Aufgabe im Dentalbereich beschrieben, bei der keine Schrumpfung toleriert werden kann. Es handelt sich um die Herstellung eines Abgusses eines Wurzelkanals. Dafür wird die Verwendung von thermoplastischen Polymeren vorgeschlagen, die in einem erwärmten und dadurch plastischen Zustand eingesetzt werden. Damit durch das erwärmte Polymer kein Schaden am natürlichen Zahnteil entsteht, werden die zu verwendenden Polymere beschränkt auf solche mit einer Erweichungstemperatur von 50 bis 70°C. Als besonders vorteilhaft wird Polycaprolacton vorgeschlagen, das eine Erweichungstemperatur von 55 bis 65° und ein Elastizitätsmodul von ca. 400 MPa hat. Offensichtlich könnte auch ein derartiges Polymer nicht eingesetzt werden für eine lasttragende Funktion, wie sie im dentalen Bereich üblich ist.

Die üblicherweise in eine Zahnwurzel eingesetzten Stifte haben einen runden Querschnitt und eine gerade Achse und es wird für den Stift in der Zahnwurzel eine entsprechende Bohrung mit einem runden Querschnitt und einer geraden Achse erzeugt. Da die Zahnwurzel weder einen runden Querschnitt noch eine gerade Achse aufweist, können Bohrung und Stift nur sehr begrenzte Abmessungen haben und muss trotzdem gegebenenfalls von der Wurzel ein nicht vernachlässigbarer Teil ausgebohrt werden, was beides der Stabilität des eingesetzten Wurzelstiftes weitere Grenzen setzt.

Die Erfindung stellt sich nun die Aufgabe, ein Präparat zur Befestigung an einem natürlichen Zahnteil oder Zahn, insbesondere zum Ersetzen eines lasttragenden Zahnteils und ein entsprechendes Verfahren zur Befestigung des Präparates an dem natürlichen Zahnteil oder Zahn, insbesondere zum Ersetzen eines lasttragenden Zahnteils durch das erfindungsgemässen Präparat zu schaffen, wobei Präparat und Verfahren es ermöglichen sollen, die oben geschilderten Schrumpfungsprobleme und die damit verbundene Beschränkung der Lebensdauer der Befestigung oder Restauration zu umgehen. Präparat und Verfahren gemäss Erfindung sollen nicht aufwendiger sein, als dies gemäss dem Stande der Technik der Fall ist, sondern sollen im Gegenteil mindestens in ausgewählten Fällen auch in dieser Hinsicht Vorteile bringen. Die Erfindung beruht auf den Erkenntnissen, dass es möglich ist, zwischen den Oberflächen eines natürlichen Zahnteils oder Zahns (Dentin und/oder Zahnschmelz) und einem Material mit thermoplastischen Eigenschaften in einem flüssigen oder mindestens plastischen Zustand formschlüssige und/oder stoffschlüssige Verbindungen zu erstellen, die den mechanischen, chemischen und biologischen Anforderungen im dentalen Bereich genügen können, dass Materialien mit thermoplastischen Eigenschaften bekannt sind, die die Festigkeitsanforderungen eines lasttragenden Zahnteils erfüllen können, und dass es möglich ist, die genannten Verbindungen mit solchen Materialien ohne unzumutbare thermische Belastung des zu restaurierenden Zahnteils zu erstellen, wenn nur ein für die Erstellung der Verbindungen notwendiges Minimum des Materials mit thermoplastischen Eigenschaften verflüssigt wird.

Zur Erstellung der genannten formschlüssigen und/oder stoffschlüssigen Verbindungen wird ein Prozess angewendet, der vom Prozess des Schweissens mit mechanischen Schwingungen (z.B. Ultraschallschweissen) abgeleitet ist. Diese Schweissprozesse beruhen im wesentlichen darauf, einen Körper aus einem Thermoplasten oder einen Körper mit Oberflächenbereichen aus einem Thermoplasten in Schwingungen zu versetzen, deren Frequenz beispielsweise im Ultraschallbereich liegt und deren Amplituden in der Grössenordnung von 1 bis 200µm liegen, und den Körper punktuell bzw. in kleinen Oberflächenbereichen (Energierichtungsgeber) mit einer weiteren Oberfläche in direkten Kontakt zu bringen. In den Kontaktbereichen entstehen im schwingenden Material Spannungskonzentrationen, die derart hoch sind, dass das Material, auch wenn es eine relativ hohe Erweichungstemperatur hat, in einen plastischen oder flüssigen Zustand gebracht wird, so dass es sich bei entsprechender Materialpaarung (z.B. zwei mischbare Thermoplasten) mit der weiteren Oberfläche verschweisst.

Das erfindungsgemässe Präparat gemäß Anspruch 1 besteht also mindestes teilweise aus einem Material mit thermoplastischen Eigenschaften und ist derart ausgelegt, dass es mechanische Schwingungen wenig dämpft, so dass das Material mittels mechanischer Schwingungen nur an Stellen, wo Spannungskonzentrationen entstehen, verflüssigt wird, wie dies beim Ultraschallschweissen der Fall ist. Die Stellen der Spannungskonzentrationen werden erzeugt durch ebenfalls aus der Technik des Ultraschallschweissens bekannte Energierichtungsgeber, die an äusseren Oberflächen (gegebenenfalls auch inneren Oberflächen) des Präparates vorzusehen oder an mit dem Präparat in Berührung stehenden Oberflächen des natürlichen Zahnteils vorzusehen oder vorhanden sind.

Die weitere Oberfläche, mit der das Material mit thermoplastischen Eigenschaften zu verbinden ist, ist eine Oberfläche eines natürlichen Zahnteils und besteht aus Dentin und/oder Zahnschmelz, die sich beide für eine Schweissverbindung in keiner Weise eignen. Diese Oberflächen weisen aber Strukturen (makroskopische, mikroskopische und/oder molekulare) auf oder werden mit derartigen Strukturen versehen, mit denen das durch die mechanischen Schwingungen verflüssigte Material in derart intensiven Kontakt kommt, dass es mit diesen Strukturen nach der Erstarrung formschlüssige und/oder stoffschlüssige Verbindungen bildet.

Für das erfindungsgemässe Präparat anwendbare Werkstoffe oder Werkstoffsysteme müssen schallleitende Eigenschaften und eine ausreichend geringe Dämpfung haben, damit das Präparat schwingungsfähig ist. Dies bedingt in Elastizitätsmodul von mindestens etwa 0,5 GPa. Der Verlustfaktor soll ausreichend hoch sein für eine Verflüssigung in den Bereichen von Spannungskonzentrationen. Es zeigt sich, dass für die erfindungsgemässen Zahn-Restaurationen thermoplastische Materialien mit Erweichungstemperaturen von bis zu ca. 350° anwendbar sind, wobei die mechanischen Schwingungen während ca. 0,1 bis 10 sec. anzuwenden sind. Dabei bleiben Wärmemenge und Expositionszeit derart gering, dass thermische Schädigungen des Zahnbeins oder des umliegenden, vitalen Gewebes nicht zu erwarten sind.

Die genannten Bedingungen bezüglich Elastizitätsmodul und Erweichungstemperatur werden von vielen Materialien mit thermoplastischen Eigenschaften (Thermoplasten oder Verbundwerkstoffe mit einer thermoplastischen Komponente, im folgenden kurz thermoplastische Materialien genannt) erfüllt, die nicht nur die mechanischen Anforderungen eines lasttragenden bzw. lastübertragenden Zahnteils erfüllen können, sondern auch in anderen medizinischen Anwendungen bereits verwendet werden.

Mindestens ein Teil der Oberfläche des erfindungsgemässen Präparates besteht aus dem thermoplastischen Material, wobei dieser Oberflächenbereich bei der Restauration mit einer Oberfläche eines zu restaurierenden Zahnteils oder eines Zahns in Berührung steht oder in Berührung bringbar ist. Ferner weist das erfindungsgemässe Präparat in den Oberflächenbereichen aus dem thermoplastischen Material gegebenenfalls Formgebungen auf, die sich als Energierichtungsgeber eignen, das heisst Kanten, Spitzen, Anformungen oder Rauhigkeiten, die sich mindestens um 0,5µm über den Rest der Oberfläche erheben. Die Funktion des Energierichtungsgeber kann aber auch durch entsprechende Formen des zu restaurierenden Zahnteils oder eines anderen Teils des Präparates übernommen werden.

Der Bereich der Präparatoberfläche, über den die Schwingungen in das Präparat eingekoppelt werden, sind vorteilhafterweise derart auszugestalten, dass dort keine Spannungskonzentrationen entstehen.

Für die Restaurierung wird das Präparat auf dem zu restaurierenden, natürlichen Zahnteil oder Zahn oder in diesem Zahnteil positioniert und dann mit geeigneten Mitteln, beispielsweise mit der Sonotrode eines Ultraschallgeräts gegebenenfalls über ein Kopplungselement in Schwingung versetzt und gegen denn natürlichen Zahnteil gepresst. Geeignete Frequenzen liegen zwischen 2 und 200 kHz (insbesondere 20, bis 80 kHz), geeignete Schwingungsamplituden zwischen 1 und 200 µm. Experimente haben gezeigt, dass mit Schwingungsleistungen von 0,2 bis 20 W pro Quadratmillimeter Wirkfläche gute Resultate erzeugt werden. Durch die auf das Präparat übertragenen Schwingungen wird das thermoplastische Material örtlich (insbesondere an seinen mit dem natürlichen Zahnteil in Berührung stehenden Oberfläche oder nahe an dieser Oberfläche) verflüssigt und mit den Oberflächen des natürlichen Zahnteils oder Zahns in einen intimen Kontakt gebracht, durch den die formschlüssige und/oder stoffschlüssige Verbindung erzeugt wird. Die optimale Abstimmung von Schwingungsamplitude und -leistung auf einen spezifischen Typus des erfindungsgemässen Präparates ist in der Regel experimentell zu bestimmen. Vorteilhafterweise werden Amplitude, Leistung und Präparat derart aufeinander abgestimmt, dass die Übertragung der Schallleistung auf die Oberfläche des Präparates optimal ist.

Die gemäss Erfindung erstellten Verbindungen zwischen Oberflächen des natürlichem Zahnteils und dem thermoplastischen Material sind insbesondere formschlüssige Verbindungen, die durch Einpressen des verflüssigten Materials in Poren und Oberflächenunebenheiten, gegebenenfalls auch Hinterschneidung des natürlichen Zahnteils entstehen. Es können aber auch stoffschlüssige Verbindungen sein, die durch adhesive oder kohäsive Austauschkräfte bedingt sind. Für die Erstellung der Verbindungen ist in jedem Falle eine intime Benetzung der natürlichen Oberflächen mit dem verflüssigten, thermoplastischen Material notwendig.

Für die Förderung der Benetzung oder für die Verbesserung der zu erstellenden Verbindungen können diese natürlichen Oberflächen auch entsprechend vorbereitet werden. Beispielsweise kann eine Schmelzoberfläche oder eine Dentinoberfläche zur Erzielung eines Formschlusses aufgerauht werden (vorteilhafte Rauhigkeit: 0,5 bis 500µm). Zur Erzielung von verbesserten formschlüssigen Verbindungen können an Dentin- oder Schmelzoberflächen auch Haltestrukturen erstellt werden, beispielsweise Gewindegänge, Furchen, Vertiefungen mit Tiefen von 0,1 bis 2mm und gegebenenfalls Hinterschneidungen. Zur Verbesserung der Benetzbarkeit der natürlichen Oberflächen mit dem thermoplastischen Material und/oder zur Erzielung von erhöhten adhesiven Austauschkräften können die natürlichen Oberflächen vor dem Einsetzen des erfindungsgemässen Präparats mit einem entsprechenden Primer vorbehandelt und/oder angeätzt werden, wobei vor oder zusammen mit einer solchen Vorbehandlung die Dentin- oder Schmelzoberflächen in an sich bekannter Weise auch versiegelt werden können. Anwendbare Primersysteme umfassen in an sich bekannter Art chemische Reaktivverbindungen, die mit dem natürlichen Zahnmaterial reagieren, und/oder molekulare Funktionalitäten welche mit entsprechenden Funktionalitäten auf der Seite des thermoplastischen Materials kooperieren. Bei diesen kooperierenden Funktionalitäten kann es sich beispielsweise um eine Abstimmung der Polaritäten der beiden Seiten zur Erhöhung der Austauschkräfte handeln, um interpenetrierende Oligomere oder um Reaktivkomponenten, die durch die Schwingungen, durch Licht, durch Wärme oder chemisch aktiviert chemische Bindungen zwischen den beiden Seiten bewirken.

Als thermoplastische Materialien für das erfindungsgemässe Präparat eignen sich beispielsweise Polyolefine, Polyacrylate, Polymetacrylate, Polyurethane, Polycarbonate, Polyamide, Polyester, Polysulfone, Polyarylketone, Polyimide, Polyphenylsulfide, Flüssig-Kristall-Polymere (liquid cristalline polymers oder LCPs), Polyacetale, halogenierte Polymere, insbesondere halogenierte Polyolefine, Polyphenylensulfide, Polysulfone, Polyether. Ebenso anwendbar sind entsprechende Copolymere oder Polymermischungen. Die genannten Thermoplasten können auch in gefülltem Zustande angewendet werden (Verbundwerkstoffe mit einer thermoplastischen Komponente), sie können also beispielsweise Fasern, Whiskers oder Partikel aus Gläsern, Keramikmaterialien, Kohlenstoff oder Polymeren enthalten, wobei die Füllung homogen sein kann oder Gradienten aufweisen kann. Entsprechende Füllsysteme sind sowohl aus der Dentaltechnik als auch aus industriellen Bereichen bekannt.

Ein Primer-System für ein Präparat, das als thermoplastischen Stoff beispielsweise ein aliphatisches Polyamid aufweist, ist einerseits für eine Umamidierung oder für eine Verbindung mit den aliphatischen Ketten des Polyamids ausgerüstet, wie dies aus der Klebetechnik bekannt ist, und andererseits, wie ein klassischer Dentalprimer, für eine Verbindung mit dem Dentin oder Zahnschmelz. Das Primersystem kann dabei aus zwei kompatiblen Primern bestehen oder die beiden Funktionen können auf einem Trägermolekül realisiert sein.

Das Material mit thermoplastischen Eigenschaften kann in an sich bekannter Art auch mit antiseptisch oder denaturierend wirkenden Agenten oder Röntgenstrahlenabsorbierenden Zusätzen ausgerüstet sein oder es kann Pigmente enthalten.

Das erfindungsgemässe Präparat hat die Form eines in einen ausgebohrten Zahn einzusetzenden Zahnteils (Zahnfüllung) oder Konstruktionselementes, einer auf einen Zahnstumpf aufzusetzenden Krone, Brücke, Teil- oder Vollprothese, Abutments oder Konstruktionselementes, eines in eine gegebenenfalls vorbearbeitete, natürliche Zahnwurzel einsetzenden Wurzelstiftes für die Befestigung eines künstlichen Zahnes, einer Brücke, eines Abutments, eines Konstruktionselementes oder einer Prothese oder die Form eines auf einen Zahn aufzusetzenden Elementes.

Das erfindungsgemässe Präparat ist dabei beispielsweise einteilig und besteht ganz aus dem thermoplastischen Material oder es weist mindestens Oberflächenbereiche aus diesem Material auf, wobei die restlichen Bereiche aus einem anderen, die Bedingungen der mechanischen Festigkeit und der Schwingfähigkeit erfüllenden Material bestehen. Dazu eignen sich in der Zahnmedizin bereits für ähnliche Zwecke verwendete, metallische oder keramische Materialien, Polymere, Verbundwerkstoffe oder Kompositwerkstoffe.

Ein erfindungsgemässes Präparat, das aus zwei verschiedenen Materialien besteht, von denen nur das eine die thermoplastischen Eigenschaften aufweist, kann auch zwei- oder mehrteilig sein, wobei ein aus dem thermoplastischen Material bestehender Präparatteil zwischen dem natürlichen Zahnteil oder Zahn und dem aus beispielsweise Keramik oder Metall bestehenden Präparatteil:positioniert wird.

Die Hauptvorteile des erfindungsgemässen Präparates und des erfindungsgemässen Verfahrens gegenüber den eingangs kurz beschriebenen, bekannten Zahn-Restaurationen sind insbesondere die markant verminderte Schrumfpung des Materials, das für die Restauration selbst in einem plastischen oder flüssigen Zustand vorliegen muss, dessen inhärente Fähigkeit zu kriechen, wodurch innere Spannungen ohne Rissbildung abgebaut werden können, und dessen inhärente Unempfindlichkeit gegen Feuchtigkeit. Diese drei Eigenschaften führen zu einer hohen Stabilität und hohen Lebensdauer der restaurierten Zähne. Ein zusätzlicher Vorteil besteht darin, dass das thermoplastische Material fähig ist, in seinem flüssigen oder plastischen zustand Ungenauigkeiten zwischen dem natürlichen Zahnteil und dem Präparat auszugleichen, was mit herkömmlichen Präparaten kaum möglich ist. Dies bedeutet, dass an das erfindungsgemässe Präparat weniger hohe Genauigkeitsansprüche gestellt werden müssen, als dies für bekannte Präparate der Fall ist, damit eine homogene Spannungsverteilung garantiert werden kann. Es ist gemäss Erfindung auch besser möglich, in einem einzigen Präparat mehrere Zahnteile (z.B. post and core) bereits vor dem Anbringen in oder an dem natürlichen Zahnteil miteinander zu verbinden, wodurch der Aufwand des Zahntechnikers relevant reduziert werden kann.

Das Verfahren weist im Vergleich zu den eingangs genannten, bekannten Verfahren weniger Verfahrensschritte auf (kein Aushärtungsschritt), benötigt weniger Zeit und ist kostengünstiger. Ein weiterer Vorteil der erfindungsgemässen Restaurationen besteht darin, dass die Aushärtung (nur Abkühlen) des thermoplastischen Materials reversibel ist. Das heisst mit anderen Worten, es sollte möglich sein, die Anwendung der mechanischen Schwingungen zur Beseitigung beispielsweise von Rissen zwischen natürlichen und künstlichen Zahnteilen oder innerhalb des künstlichen Zahnteiles zu wiederholen oder gegebenenfalls fehlende Teile mittels Ultraschallschweissen zu ergänzen. Dies bedeutet, dass die erfindungsgemässe Restauration in sehr einfacher Weise revidiert oder repariert werden kann.

Das Verfahren und beispielhafte Ausführungsformen des erfindungsgemässen Präparates werden im Zusammenhang mit den folgenden Figuren im Detail beschrieben. Die in den vorgehenden Abschnitten beschriebenen Bedingungen und weiteren Merkmale des erfindungsgemässen Präparates sind für alle Ausführungsformen entsprechend anwendbar. Dabei zeigen:
- **Figur 1**: vier beispielhafte Ausführungsformen von erfindungsgemässen Präparaten zur Füllung eines ausgebohrten Zahnes (Längsschnitt);
- **Figur 2**: einen mit einem erfindungsgemässen Präparat gemäss Figur 1 gefüllten Zahn (Längsschnitt);
- **Figur 3**: die Aufkronung eines Zahnstumpfes mit Hilfe eines erfindungsgemässen Präparates (Längsschnitt);
- **Figur 4**: eine Ausführungsform des erfindungsgemässen Präparates in der Form eines Wurzelstiftes (Längsschnitt), der in eine entsprechend vorbereitete, natürliche Zahnwurzel eingesetzt wird;
- **Figur 5**: eine weitere, beispielhafte Ausführungsform des erfindungsgemässen Präparates, die im Sinne eines Wurzelstiftes für die Befestigung eines künstlichen Zahns, einer Brücke, eines Abutments, eines Konstruktionselementes oder einer Prothese an einer natürlichen Zahnwurzel ausgerüstet ist (Längsschnitt);
- **Figuren 6 und 7**: zwei beispielhafte Ausführungsformen der distalen Enden des erfindungsgemässen Präparates gemäss Figur 5;
- **Figur 8**: eine weitere, beispielhafte Ausführungsform des erfindungsgemässen Präparats in der Form eines künstlichen Zahnes, der auf eine natürliche Zahnwurzel aufgesetzt wird (Längsschnitt);
- **Figur 9**: eine Ausführungsform eines Präparates in Form eines auf einen Zahn aufsetzbaren Schmuckelementes.

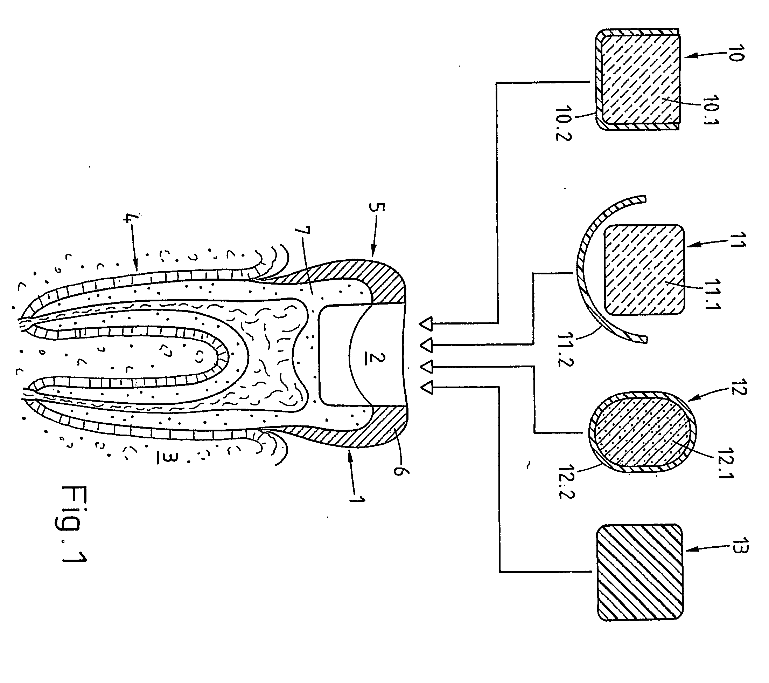

**Figur 1** zeigt als Beispiel einer Vielzahl von Zahnkavitäten, die nach dem erfindungsgemässen Verfahren gefüllt oder zur Befestigung eines Konstruktionselementes verwendet werden können, einen beispielsweise wegen Karies ausgebohrten Zahn 1, der eine nach dem erfindungsgemässen Verfahren zu füllende Öffnung 2 aufweist. Der Zahn 1 ist entlang seiner Längsachse geschnitten dargestellt. Er weist eine in das Knochengewebe 3 des Kieferknochens eingewachsene Wurzel 4 und eine über den Kieferknochen ragende Krone 5 auf, die mit Schmelz 6 überzogen ist. Das tragende Element des Zahnes ist das Zahnbein 7, das aus porösem Dentin besteht. In seinem Inneren weist das Zahnbein 7 eine Zahnhöhle auf, die mit durchblutetem und von einem Nerv versorgtem Bindegewebe (Pulpa) gefüllt ist. Die Öffnung 2 reicht durch den Zahnschmelz 6 in das Zahnbein 7, das heisst, es weist eine zugängliche Oberfläche innerhalb der Öffnung 2 auf, die eine offenporige Struktur hat. Zusätzlich kann sowohl die Dentinoberfläche als auch die Schmelzoberfläche in der Öffnung für eine Verbesserung der Verbindung zwischen natürlichem Zahnteil und einzusetzendem Präparat vorbehandelt werden (z.B. durch Aufrauhen, Strukturieren, Anätzen und/oder Primerbehandlung).

Für die Restauration des in der Figur 1 dargestellten, ausgebohrten Zahns 1 durch Füllen der Öffnung 2 wird gemäss Erfindung beispielsweise eines der über dem ausgebohrten Zahn 1 dargestellten Präparate 10, 11, 12 oder 13 verwendet.

Das Präparat 10 weist einen Körper 10.1 aus einem an sich für Füllkörper bekannten Material auf und eine Kontaktschicht 10.2, die aus dem thermoplastischen Material besteht und die die Oberfläche des Körpers 10.1 vollständig oder teilweise bedeckt. Beispielsweise ist diejenige Präparatseite, die in der Öffnung 2 gegen aussen gewandt ist, frei von thermoplastischem Material. Das Präparat 10 ist derart dimensioniert, dass es im wesentlichen ohne Kraftanwendung mindestens teilweise in der Öffnung 2 positionierbar ist.

Das in der Öffnung 2 positionierte Präparat 10 wird dann mit Hilfe des Resonators (nicht dargestellt) einer mechanische Schwingungen erzeugenden Vorrichtung, beispielsweise mit Hilfe der Sonotrode eines Ultraschallgerätes mit mechanischen Schwingungen angeregt und gleichzeitig in die Öffnung 2 gepresst. Gegebenenfalls wird zwischen Sonotrode und Präparat ein Kopplungselement verwendet, das auf seiner dem Präparat zugewandten Seite in der Art eines Negativs der Präparatoberfläche ausgebildet ist und auf der der Sonotrode zugewandten Seite eine bezüglich Grösse auf eine Standard-Sonotrode ausgerichtete, ebene Fläche aufweist. Dadurch verflüssigt sich das Material der Kontaktschicht 10.2 mindestens teilweise und wird durch den angewendeten Druck mit Zahnschmelz 6 und Zahnbein 7 in intimen Kontakt gebracht, der zu formschlüssigen und adhäsiven Verbindungen führt. Gleichzeitig wird das Präparat gegebenenfalls tiefer in die Öffnung 2 geschoben. Wenn der Präparatkörper 10.1 genügend in die Öffnung 2 geschoben ist und nicht mehr des thermoplastischen Materials in Poren und Unebenheiten gepresst werden kann (beispielsweise zu erkennen am Herauspressen des Materials zwischen Zahnschmelz und Körper 10.1), wird die Schwingungsanregung gestoppt. Dabei wird vorteilhafterweise der Pressdruck aufrechterhalten, bis das thermoplastische Material wieder erstarrt ist. Nach der Erstarrung des thermoplastischen Materials kann der Körper 10.1 entsprechend dem Stand der zahnärztlichen Technik an die Form des ursprünglichen Zahnes 1 angepasst und dabei gegebenenfalls die Kontaktschicht 10.2 an dieser Stelle vom Präparatkörper 10.1 entfernt werden.

Das ebenfalls für die Füllung der Öffnung 2 des Zahnes 1 geeignete Präparat 11 unterscheidet sich vom Präparat 10 lediglich dadurch, dass es anstelle einer am Körper befestigten Kontaktschicht eine separat vom Körper 11.1 anwendbare Kontaktfolie 11.2 aufweist, die aus dem Material mit thermoplastischen Eigenschaften besteht. Die Kontaktfolie 11.2 wird vor der Positionierung des Körpers 11.1 oder gleichzeitig mit dem Körper 11.1 in die Öffnung 2 eingebracht.

Das Präparat 12 weist einen Präparatkörper 12.1 auf aus einem hochviskosen Kompositmaterial (durch z.B. Licht, Wärme oder Ultraschall aushärtbarer Zement oder Ormocer-artiges System), wie sie in der Zahnmedizin bekannt sind. Der Präparatkörper 12.1 ist in einer vorteilhafterweise flexiblen Kontaktschicht 12.2 aus dem thermoplastischen Material eingeschlossen. Das Material der Präparatkörpers 12.1 und der Kontaktschicht 12.2 sind dabei derart aufeinander abgestimmt, dass zwischen ihnen eine Verbindung entsteht (z.B. durch Silanisierung, Oberflächenaktivierung, Pfropfpolymerisation reaktiver Gruppen auf der Seite der Kontaktschicht).

Der Vorteil des Präparates 12 gegenüber den Präparaten 10 und 11 besteht darin, dass es in einem grösseren Ausmass an die Form der Öffnung 2 anpassbar ist. Der durch den Kompositwerkstoff eingehandelte Nachteil der Schrumpfung wird wettgemacht durch die Verbindung zwischen dem ausgehärteten Kompositwerkstoff und dem thermoplastischen Material, die dazu führt, dass durch Schrumpfung des Zements entstehende Spannungen auf das thermoplastische Material übertragen werden und durch Relaxation abgebaut werden können.

Die Kontaktschicht 12.2 kann auch relativ steif und oben offen sein, wobei sie vom Zahnarzt mit dem Zement befüllbar ist. In diesem Falle ist es vorteilhaft, für die Anwendung der Schwingungen den Zement mit einem Deckelement (z.B. Teflonfolie, Metallfolie, Metallelement etc.) abzudecken.

Die Kontaktschichten 10.2 oder 12.2 oder die Kontaktfolie 11.2 haben vorteilhafterweise eine Dicke von ca. 0,01 bis 1 mm. Es ist in keiner Weise notwendig, dass die Kontaktschicht oder -folie an allen Stellen eine gleiche Dicke aufweist und die mit dem natürlichen Zahnteil in Berührung kommenden Oberflächen vollständig überdeckt. Vorteilhafterweise weist sie an ihrer gegen das Zahnbein gerichteten Oberfläche Energierichtungsgeber in Form von um mindestens 1µm vorstehenden Rippen, Pyramiden, Kegel, Halbkugeln etc. auf. Die Funktion der Energierichtungsgeber kann aber auch von der Dentin/Schmelz-Oberläche übernommen werden. Im Falle eines Präparates 11 mit einer Kontaktfolie 11.2 ist es auch möglich, die Oberfläche des Präparatkörpers 11.1 mit den Energierichtungsgebem auszustatten.

Das thermoplastische Material der Kontaktschichten 10.2 oder 12.2 oder der Kontaktfolie 11.2 kann, wie bereits weiter oben erwähnt auch Reaktivharze enthalten, die mit dem natürlichen Zahnmaterial oder mit einem darauf aufgetragenen Primer chemische Verbindungen bildend reagieren.

Das Präparat 13, das sich ebenfalls für die Füllung der Öffnung 2 des Zahnes 1 eignet, weist keinen von einer Kontaktschicht oder -folie umgebenen Präparatkörper aus einem Material ohne thermoplastische Eigenschaften auf, sondern besteht ganz aus einem thermoplastischen Material, vorteilhafterweise aus einem gefüllten Thermoplasten, wobei der Füllungsgrad vom Zentrum gegen die mit den Zahnoberflächen zu verbindenden Präparatoberflächen abnehmen kann. Das thermoplastische Material kann zusätzlich für Zahn-Füllkörper gemäss dem Stande der Technik bekannte Pigmente enthalten. Auch das Präparat 13 ist zu einem etwas höheren Grad an die Form der Öffnung 2 anpassbar, als die Präparate 10 und 11, denn die zur Verfügung stehende Menge an thermoplastischem Material ist grösser.

Das Präparat 13 eignet sich insbesondere für die Befestigung eines Konstruktionselementes, also eines Unterbauelementes zur Befestigung einer Prothese. Es weist dafür beispielsweise an derjenigen Seite, die nach dem Einsetzen an der Zahnoberfläche liegt, eine Bohrung auf, in die das Konstruktionselement vor oder nach dem Einsetzen des Präparates eingeschweisst wird.

Die Kavität 2 kann, wie oben beschrieben, mit einem der Präparate 10 bis 13 gefüllt werden. Sie kann aber auch mit einer Mehrzahl gleicher oder verschiedener Präparate gefüllt werden, wobei dann ein erstes Präparat eingesetzt und mit mechanischen Schwingungen beaufschlagt, dann ein zweites Präparat eingesetzt und mit mechanischen Schwingungen beaufschlagt wird, und so fort.

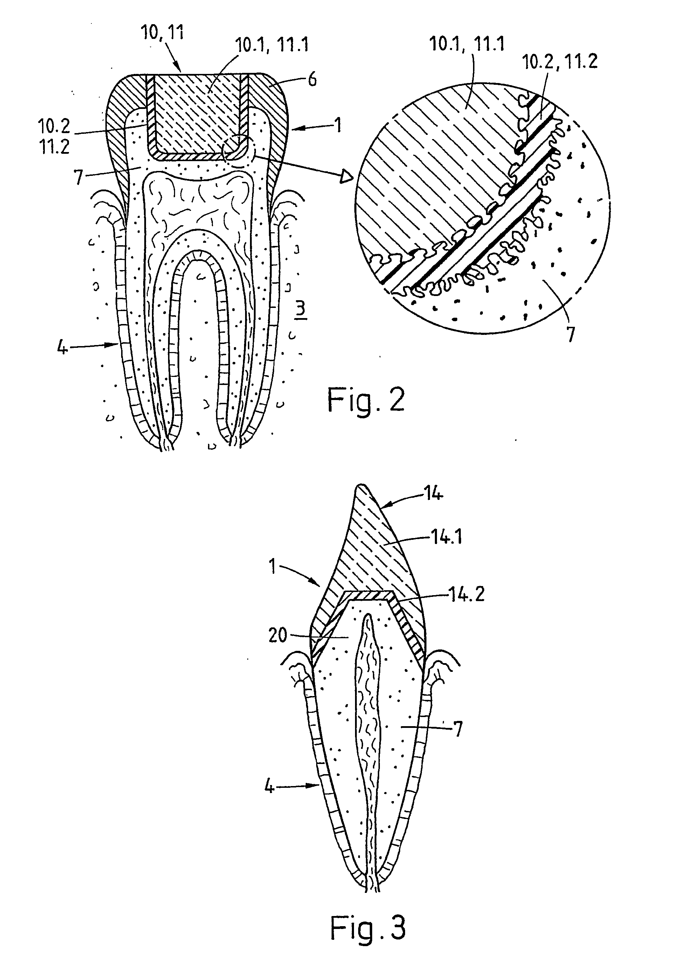

Für den Fall, dass die Öffnung 2 nicht wie in der Figur 2 dargestellt nur stirnseitig zugänglich ist sondern auch von der Zahnseite, wird für das Einsetzten der Präparate 10 bis 13 wie für Füllverfahren gemäss dem Stande der Technik eine Hilfskonstruktion verwendet, z.B. eine Manschette um den Zahn gelegt und nach der Füllung wieder entfernt. Diese Hilfskonstruktion hat eine Innenoberfläche, an der sich das thermoplastische Material nicht verflüssigt oder von der es leicht trennbar ist. Am fertig eingesetzten Präparat wird auf der offenen Seite die Kontaktschicht oder -folie entfernt.

**Figur 2** zeigt denselben Zahn 1 wie Figur 1 aber nach der Füllung der Öffnung 2 mit dem Präparat 10 oder 11 und nach einer gegebenenfalls notwendigen Anpassung der Aussenfläche an die ursprüngliche Form des Zahns. Insbesondere ist in einem grösseren Massstab ein Teil der Kontaktschicht 10.2 oder Kontaktfolie 11.2 nach Verflüssigung und Einpressung in die Poren des Zahnbeins 7 dargestellt. Aus diesem Detail ist der durch die Verflüssigung und Einpressung des thermoplastischen Materials in Poren oder Oberflächenunebenheiten des Dentins und gegebenenfalls Zahnschmelzes erzeugte Formschluss zwischen natürlichem Zahnteil und Präparat zu erkennen. Dieser Formschluss sorgt für eine sehr stabile Verankerung des Präparats. Wie ebenfalls aus dem vergrössert dargestellten Detailbereich zu erkennen ist, ist es vorteilhaft, die Oberfläche des Präparatkörpers 10.1 oder 11.1 derart auszugestalten, dass auch hier ein Formschluss (oder auch adhesive Verbindung) zwischen dem Material des Körpers und dem thermoplastischen Material möglich wird. Dafür kann die Oberfläche des Präparatkörpers 10.1 oder 11.1 an denjenigen Seiten, an denen er vom thermoplastischen Material umgeben wird, entsprechend rauh ausgestaltet (Rauheit von ca. 0,5 bis 50µm) oder mit einer entsprechenden Oberflächenstruktur versehen werden. Es ist auch denkbar, die entsprechenden Oberflächenbereiche des Präparatkörpers 10.1 oder 11.1 aus einem porösen, beispielsweise gesinterten Material herzustellen (Poren mit Grössen von 10 bis 300µm, Porosität von 2 bis 80%).

**Figur 3** zeigt als weitere Ausführungsform des erfindungsgemässen Präparates eine künstliche Krone 14, die erfindungsgemäss auf einem natürlichen Zahnstumpf 20 aufgebracht ist. Die Krone 14 weist einen Kronenkörper 14.1 aus einem üblicherweise für diesen Zweck verwendeten Material (z.B. Zahnlegierungen, Kunststoffe, Komposite, Keramische Verbindungen) auf. Zwischen diesem und dem Zahnbein 7 befindet sich wiederum eine Kontaktschicht 14.1, die aus einem thermoplastischen Material besteht und die als Beschichtung des Kronenkörpers 14.1 (analog zur Kontaktschicht 10.1 des Präparats 10) oder als separate Kontaktfolie (analog zur Kontaktfolie 11.2 des Präparats 11) eingebracht wurde und die mittels mechanischer Schwingungsanregung und Pressung mit dem Zahnbein 7 des Zahnstumpfs verbunden und gegebenenfalls auch in einer entsprechenden Oberflächenstruktur des Kronenkörpers 14.1 verankert ist, wie dies in der Detaildarstellung der Figur 2 dargestellt ist. Das Präparat 14 kann nicht nur eine Krone, sondern in der gleichen Weise eine Brücke, ein Abutment, ein Konstruktionselement oder eine Prothese oder Teilprothese sein.

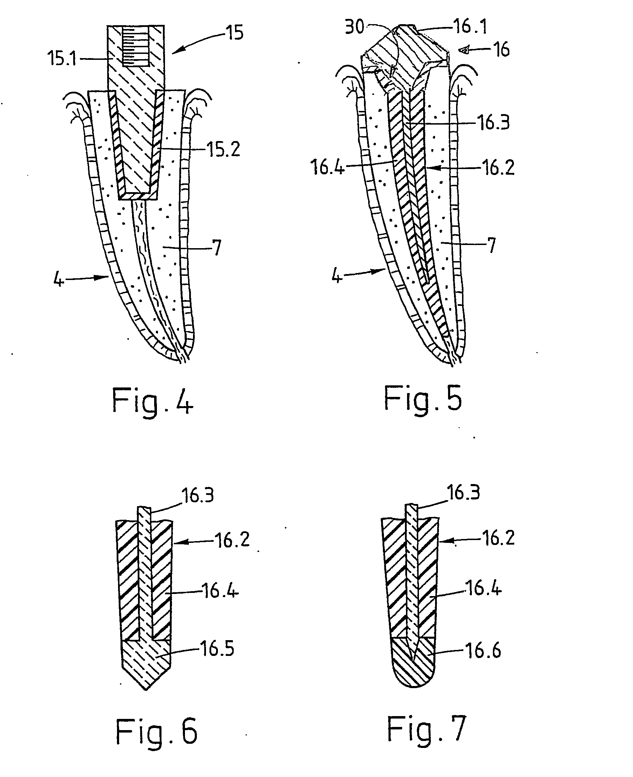

**Figur 4** zeigt eine weitere Ausführungsform 15 des erfindungsgemässen Präparates, das die Form und Funktion eines Befestigungskörpers für eine Krone, Brücke, Abutment, Konstruktionselement oder Prothese (z.B. Wurzelstift) hat und in eine entsprechend vorbereitete (ausgebohrte) Zahnwurzel eingesetzt wird, um daran einen künstlichen Zahn, eine Brücke, ein Abutment, ein Konstruktionselement oder eine Teil- oder Vollprothese zu befestigen. Das Präparat weist wiederum einen Präparatkörper 15.1 und eine Kontaktschicht 15.2 oder Kontaktfolie auf, kann aber auch ganz aus dem thermoplastischen Material bestehen. Das Präparat 15 ist in derselben Weise ausgestattet wie die Zahnfüllungen 10, 11 oder 13 gemäss Figuren 1 und 2. Die Beschreibung dieser Figuren ist entsprechend anwendbar. An seinem proximalen Ende ist das Präparat mit Befestigungsmitteln versehen, beispielsweise wie dargestellt mit einem Sackloch mit Innengewinde. Selbstverständlich kann ein derartiges Befestigungsmittel auch erst nach dem Einsetzen in die Zahnwurzel erstellt werden.

**Figur 5** zeigt ein weiteres Beispiel 16 eines erfindungsgemässen Präparates. Es ist wiederum ein Präparat, das der Befestigung eines weiteren, künstlichen Zahnteiles (Wurzelstift) dient und dazu einen Befestigungskörper 16.1 aufweist. Der Befestigungskörper 16.1 besteht aus einem für die lasttragende Funktion geeigneten Material, beispielsweise aus Titan. Am Befestigungskörper ist ein Wurzelteil 16.2 angeordnet, der einen vorteilhafterweise elastisch oder plastisch deformierbaren und dadurch an einen spezifischen Wurzelkanal anpassbaren Kern 16.3, beispielsweise einen Titandraht und eine den Kern 16.3 umhüllenden Hülle 16.4 aus dem thermoplastischen Material aufweist. Der Wurzelteil 16.2 wird mindestens teilweise in der Zahnhöhle der entsprechend vorbereiteten, für den Befestigungskörper gegebenenfalls auch ausgebohrten, natürlichen Zahnwurzel 4 positioniert, wobei er sich an die Form dieser Höhle anpasst. Dann wird der über die Zahnwurzel 4 vorstehende Befestigungskörper 16.1 mit mechanischen Schwingungen beaufschlagt und gegen die Zahnwurzel 4 gepresst, so dass er in einen entsprechend auf der Oberfläche der Zahnwurzel 4 vorbereiteten Sitz 30 zu sitzen kommt und der Wurzelteil möglichst tief in die Höhle der Zahnwurzel geschoben wird. Gleichzeitig wird das Hüllenmaterial mindestens teilweise verflüssigt und dadurch die Hüllenform an die Kanalform angepasst und das thermoplastische Material mit dem Zahnbein verbunden.

Das Präparat 16 kann für eine Anwendung auf der Wurzel eines Backezahnes auch mit mehreren Wurzelteilen 16.2 ausgerüstet sein. Insbesondere in dieser Anwendung ist es vorteilhaft, das thermoplastische Material der Hülle 16.4 mit antiseptisch oder denaturierend wirkenden Agenten auszurüsten.

Figur 5 zeigt das Präparat 16 in seinem montierten Zustand, das heisst nach der Behandlung mit den mechanischen Schwingungen. Der Befestigungskörper sitzt in seinem Sitz 30. Der Kern 16.3 des Wurzelteils 16.2 sitzt in der Höhle der Zahnwurzel 4 umgeben von der Hülle 16.4, deren thermoplastisches Material mindestens teilweise in die Poren und Oberflächenunebenheiten des Zahnbeins 7 eingepresst und so in diesem verankert ist. Durch eine geeignete Oberflächenstruktur des Kerns 16.3 (nicht dargestellt) ist auch für eine gute Verankerung zwischen Kern 16.3 und Hülle 16.4 gesorgt.

Für das Präparat gemäss Figur 5 ist es besonders vorteilhaft, das thermoplastische Material der Hülle 16.4 antibiotisch oder antiseptisch auszurüsten und dadurch die negativen Effekte von im Wurzelkanal verbliebenen Resten der Pulpa auszuschalten.

Kern 16.3 und Hülle 16.4 können auch beide aus dem thermoplastischen Material bestehen, wobei eine verstärkende Füllung im Kernbereich einen höheren Anteil als im Hüllenbereich haben kann.

**Figuren 6 und 7** zeigen zwei Varianten des distalen Endes des Präparates 16 gemäss Figur 5, mit denen verhindert werden kann, dass der Wurzelteil 16.2 zu tief in die Wurzelhöhle vorgeschoben werden kann und/oder dass von dem verflüssigten Material aus der Wurzelhöhle ausgepresst werden kann. Gemäss Figur 6 weist der Kern 16.3 eine Verdickung 16.5 auf, die den Vorschub im Wurzelkanal limitiert und/oder als Dichtung dienen kann und die auch die Position des Wurzelteils 16.2 im Röntgenbild gut sichtbar macht. Gemäss Figur 7 ist am distalen Ende des Präparates ein Stopfen 16.6 aus einem weichen Material, beispielsweise aus Guttapercha, angeordnet. Der Stopfen 16.6 wird gegebenenfalls durch die Schwingungen des Präparates ganz in einen plastischen Zustand gebracht und kann so die innere Öffnung des Wurzelkanals abdichten.

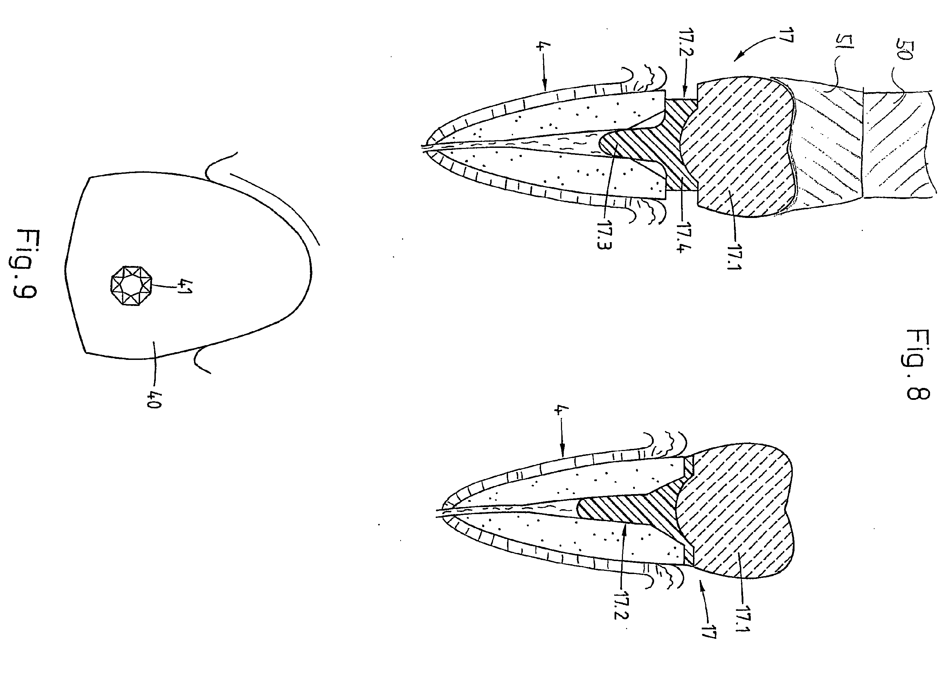

**Figur 8** zeigt eine weitere Ausführungsform 17 des erfindungsgemässen Präparates. Diese weist die Form eines auf eine natürliche Zahnwurzel 4 aufzusetzenden, künstlichen Zahnes auf. Das Präparat 17 hat einen Kronenteil 17.1 und einen Verbindungsteil 17.2, wobei der Kronenteil 17.1 aus einem für einen künstlichen Zahn geeigneten Material und der Verbindungsteil 17.2 mindestens teilweise aus dem thermoplastischen Material besteht. Der Verbindungsteil 17.2, der am Kronenteil 17.1 befestigt sein oder von diesem unabhängig eingesetzt werden kann, ist beispielsweise als flache Platte oder Folienstück ausgebildet oder weist, wie dargestellt, einen mindestens teilweise in der Höhle der Zahnwurzel 4 positionierbaren Wurzelfortsatz 17.3 auf, der an eine auf der Wurzeloberfläche positionierbaren Verbindungsplatte 17.4 angeformt ist. Die Verbindungsplatte 17.4 ist ihrerseits mit dem Kronenteil 17.1 formschlüssig verbunden oder wird bei der Montage mit diesem formschlüssig verbunden. Das Präparat 17 ist oben in der Figur 8 auf der Zahnwurzel 4 positioniert, also vor der Anwendung der mechanischen Schwingungen, dargestellt und unten im fertig montierten Zustand, also nach der Anwendung der mechanischen Schwingungen.

Für die Montage wird das Präparat 17 derart auf der vorbereiteten Zahnwurzel 4 positioniert, dass der Wurzelfortsatz 17.3 mindestens teilweise in der Höhle der Zahnwurzel 4 positioniert ist. Dann wird das Präparat beispielsweise mit der Sonotrode 50 eines Ultraschallgerätes mit Schwingungen beaufschlagt. Gegebenenfalls kann zwischen Sonotrode 50 und Kronenteil 17.1 ein Kopplungselement 51 positioniert werden, wobei das Kopplungselement 51 einerseits an den Kronenteil 17.1 und andererseits an die Sonotrode 50 angepasst ist. Zur Positionierung von Sonotrode 50, Kopplungselement 51 und Kronenteil 17.1 können diese lösbar beispielsweise mittels Vakuum oder mittels Klebstoff aneinander haften.

Im Falle eines zweiteiligen Präparats werden zuerst beide Teile positioniert und dann das Ganze mit Schwingungen beaufschlagt, wobei das thermoplastische Material des Verbindungskörpers 17.2 lokal, insbesondere an dessen Berührungsstellen mit dem Zahnbein 7, verflüssigt und gleichzeitig weiter in die Höhle der Zahnwurzel 4 geschoben wird. Ebenfalls gleichzeitig wird das thermoplastische Material des Verbindungskörpers 17.2 auch an dessen Berührungsfläche mit dem Kronenteil 17.1 verflüssigt und in entsprechend vorgesehenen Oberflächenstrukturen eingepresst, wie dies im Zusammenhang mit der Figur 2 beschrieben wurde. Es ist auch möglich, zuerst den Verbindungskörper 17.2 zu positionieren und unter Anwendung der Schwingungen mit der Zahnwurzel zu verbinden und erst dann den Kronenteil 17.1 zu positionieren und diesen dann unter nochmaliger Anwendung der Schwingungen mit dem Verbindungskörper 17.2 zu verbinden.

In analoger Weise, wie in der Figur 8 für einen auf eine natürliche Zahnwurzel aufzusetzenden Kronenteil dargestellt und beschrieben, kann auch ein anderer, künstlicher Zahnteil auf eine eigens dafür geschaffene Kavität in einem natürlichen Zahnteil aufgesetzt werden.

**Figur 9** zeigt einen vollständigen, natürlichen Zahn (40), auf dem ein Schmuckelement (41), beispielsweise ein Brillant befestigt ist, wobei die Hinterseite des Schmuckelementes im Sinne der Erfindung eine Schicht eines Materials mit thermoplastischen Eigenschaften trägt oder eine entsprechende Folie bei der Befestigung zwischen Zahn und Schmuckelement positioniert wird. In derselben Weise sind auch andere Elemente wie beispielsweise Verblendungen und Fixierelemente für beispielsweise in einer Zahnkorrektur verwendete Drähte an Zähnen befestigbar.

## Patentansprüche

1. Präparat in der form einer Zahnfüllung, eines Konstruktionselementes, einer Krone, Brücke, Teil- oder Vollprothese, eines Abutments oder eines Wurzelstiftes. (10, 11, 12, 13, 14, 15, 16, 17) zur Verwendung in einem Verfahren zur Befestigung an einem natürlichen Zahnteil oder Zahn, insbesondere zum Ersatz eines lasttragenden Zahnteils, in welchen Verfahren ist, in einem ersten Schritt ein natürlicher Zahnteil oder Zahn vorbereitet wird, in einem zweiten Schritt das Präparat (10, 11, 12, 13, 14, 15, 16, 17) derart im Bereiche des vorbereiteten Zahnteils oder Zahns positioniert wird, dass das Material mit thermoplastischen Eigenschaften mit einer Dentinoberfläche und/oder einer Schmelzoberfläche des natürlichen Zahnteils oder Zahns in Berührung steht oder in Berührung bringbar ist, und in einem dritten Schritt das Präparat zu mechanischen Schwingungen angeregt und gleichzeitig gegen den Zahnteil oder Zahn gepresst wird, wobei das Präparat zur Anregung mit mechanischen Schwingungen und zur Pressung mit einer Sonotrode (50) eines Ultraschallgerätes oder mit der Sonotrode (50) und einem zwischen Sonotrode (50) und Präparat positionierten Kopplungselement (51) gegen den natürlichen Zahnteil oder Zahn gepresst wird, und wobei im dritten Schritt mechanische Schwingungen mit einer Frequenz im Bereiche zwischen 2 und 200 kHz verwendet werden, wobei das Präparat mindestens einen Bereich (10.2,12.2,14.2, 15.2,16.2,17.2) oder einen Teil (11.2) aus einem Material mit thermoplastischen Eigenschaften aufweist, wobei dieses Material mindestens einen Teil der Präparatoberfläche bildet oder mindestens, einen Teil der Präparatoberfläche bildend positionierbar ist, wobei das Präparat Schwingungseigenschaften mit derart kleinen Dämpfungsverlusten aufweist, dass für eine Verflüssingung des Materials mit thermoplastischen Eigenschaften mittels Schwingungen lokale Spannungskonzentrationen notwendig sind, wobei das Präparat derart ausgestaltet ist, dass Spannungskonzentrationen nur im Bereich der Präparatoberfläche auftreten, und wobei das Material mit thermoplastischen Eigenschaften ein Elastizitätsmodul aufweist, das grösser ist als 0,5 GPa.

2. Präparat (10, 11, 12, 13, 14, 15, 16, 17) nach Anspruch 1, **dadurch gekennzeichnet, dass** das Material mit thermoplastischen Eigenschaften ein thermoplastisches Material oder ein Verbundwerkstoff mit einer thermoplastischen Komponente ist.

3. Präparat (10, 11, 12, 13, 14, 15, 16, 17) nach Abspruch 2, **dadurch gekennzeichnet, dass** das thermoplastische Material oder die thermoplastische KomHilfsantreg 1 ponente ein Polyolefin, ein Polyacrylat, ein Polymetacrylat, ein Polyurethan, ein Polycarbonat, ein Polyamid, ein Polyester, ein Polysulfon, ein Polyarylketon, ein Polyimid, ein Polyphenylsulfid, ein Flüssig-Kristall-Polymer, ein Polyacetal, ein halogeniertes Polymer, insbesondere ein halogeniertes Polyolefin, ein Polyphenylensulfid, ein Polysulfon oder ein Polyether oder ein entsprechendes Copolymer oder eine Mischung aus mindestens zwei der genannten Polymere ist.

4. Präparat nach einem der Ansprüche 1 bis 3, **dadurch gekennzeichnet, dass** das Material mit thermoplastischen Eigenschaften ein Thermoplast mit, Beimischungen von Fasern; Whiskers, Partikeln, antiseptisch oder denaturieren wirkenden Agentien oder Röntgenstrahlen-absorbierende Bestandteilen ist.

5. Präparat nach einem der Ansprüche 1 bis 3, **dadurch gekennzeichnet, dass** das Material mit thermoplastischen Eigenschaften eine mit einer Oberfläche des natürlichen Zahnteils oder mit einem darauf aufgebrachten Primer reagierende Aktivkomponente aufweist.

6. Präparat (10, 11, 12, 14, 15, 16, 17) nach einem der Ansprüche 1 bis 5, **dadurch gekennzeichnet, dass** es einen weiteren Bereich (10.1, 12.1, 14.1, 15.1, 16.1, 17.1) oder Teil (11.1) aus einem metallischen oder keramischen Material, aus einem Polymer oder aus einem Verbund- oder Kompositwerkstoff aufweist.

7. Präparat (10, 11, 12, 13) nach einem der Ansprüche 1 bis 6, **dadurch gekennzeichnet, dass** es die Form einer in eine Zahnkavität (2) einzubringenden Zahnfüllung oder eines auf einem Zahn (40) zu befestigenden Elementes (41) hat.

8. Präparat (10, 11, 12) nach Anspruch 6, **dadurch gekennzeichnet, dass** es einen Präparatkörper (10.1, 11.1, 12.1) und eine am Präparatkörper (10.1, 11.1, 12.1) angeordnete Kontaktschicht (10.2,12.2) oder eine einen separaten Präparatteil bildende Kontaktfolie (11.2) aufweist.

9. Präparat (13) nach Anspruch 6, **dadurch gekennzeichnet, dass** es ganz aus dem Material mit thermoplastischen Eigenschaften besteht.

10. Präparat (14) nach einem der Ansprüche 1 bis 6, **dadurch gekennzeichnet, dass** es auf einem natürlichen Zahnstumpf (20) aufsetzbar ist, und dass das Material mit den thermoplastischen Eigenschaften eine innere Oberfläche von Krone, Abutment, Konstruktionselement, Brükke oder Prothese bildet oder darauf positionierbar ist.

11. Präparat (15,16) nach einem der Ansprüche 1 bis 6, **dadurch gekennzeichnet, dass** es einen in einer Kavität eines natürlichen Zahnteils einsetzbaren Befestigungskörper (15.1, 16.1) für die Befestigung eines weiteren, künstlichen Zahnteils aufweist.

12. Präparat (16) nach Anspruch 11, **dadurch gekennzeichnet, dass** es mindestens einen am Befestigungskörper (16.1) angeordneten Wurzelteil (16.2) aufweist mit einem elastisch oder plastisch deformierbaren Kern (16.3) und eine um den Kern (16.3) angeordnete Hülle (16.4) aus dem Material mit thermoplastischen Eigenschaften.

13. Präparat (16) nach Anspruch 12, **dadurch gekennzeichnet, dass** am distalen Ende des Kerns (16.3) eine Verdickung (16.5) aus dem Kernmaterial oder ein Stopfen (16.6) aus einem weichen Thermoplasten angeordnet ist.

14. Präparat (17) nach einem der Ansprüche 1 bis 6, **dadurch gekennzeichnet, dass** es einen Kronenteil (17.1) und einen Verbindungskörper (17.2) aufweist, wobei der Verbindungskörper (17.2) mindestens teilweise aus dem Material mit thermoplastischen Eigenschaften besteht und derart ausgestaltet ist, dass er mindestens teilweise in einer Kavität eines natürlichen Zahnteils positionierbar ist.

15. Nicht-therapeutisches Verfahren zum Befestigen eines Präparates das präparat mindestens einen Bereich (10.2, 12.2, 14.2, 15.2, 16.2, 17.2), oder einen Teil (11.2) aus einem Material mit thermoplastischen Eigenschaften aufweist wobei dieses Material mindestens einen Teil der Präparatoberfläche bildet oder mindestens einen Teil der Präparatoberfläche bildend positionierbar ist, und Schwingungseigenschaften mit derart kleinen Dämpfungsverlusten aufweist, dass für eine Verflüssigung des Materials mit thermoplastischen Eigenschaften mittels Schwingungen lokale Spannungskonzentrationen notwendig sind, wobei das Präparat derart ausgestaltet ist, dass Spannungskonzentrationen nur im Bereich der Präparatoberfläche auftreten, an einem natürlichen Zahnteil oder Zahn, **dadurch gekennzeichnet, dass** in einem ersten Schritt ein natürlicher Zahnteil oder Zahn vorbereitet wird, dass in einem zweiten Schritt das Präparat (10, 11, 12, 13, 14, 15, 16, 17) derart im Bereiche des vorbereiteten Zahnteils oder Zahns positioniert wird, dass das Material mit thermoplastischen Eigenschaften mit einer Dentinoberfläche und/oder einer Schmelzoberfläche des natürlichen Zahnteils oder Zahns in Berührung steht oder in Berührung bringbar ist, und dass in einem dritten Schritt das Präparat zu mechanischen Schwingungen angeregt und gleichzeitig gegen den Zahnteil oder Zahn gepresst wird, wobei das Präparat zur Anregung mit mechanischen Schwingungen und zur Pressung mit einer Sonotrode (50) eines Ultraschallgerätes oder mit der Sonotrode (50) und einem zwischen Sonotrode (50) und Präparat positionierten Kopplungselement (51) gegen den natürlichen Zahnteil oder Zahn gepresst wird, wobei im dritten Schritt mechanische Schwingungen mit einer Frequenz im Bereiche zwischen 2 und 200 kHz verwendet werden, und wobei dass das Material mit thermoplastischen Eigenschaften ein Elastizitätsmodul aufweist, das grösser ist als 0,5 GPa.

16. Verfahren nach Anspruch 15, **dadurch gekennzeichnet, dass** im ersten Schritt Dentin- und/oder Schmelzoberflächen aufgerauht, mit Haltestrukturen versehen und/oder mit einem. Primer und/oder einem Versiegelungsmittel vorbehandelt werden.

17. Verfahren nach einem der Ansprüche 15 oder 16, **dadurch gekennzeichnet, dass** ein zweiteiliges Präparat (11) verwendet wird und dass im zweiten Schritt zuerst ein erster Präparatteil (11.2), der das Material mit den thermoplastischen Eigenschaften aufweist, positioniert und mit den Schwingungen beaufschlagt wird und dann ein zweiter Präparatteil (12.1) positioniert und mit den Schwingungen beaufschlagt wird.

## Claims

1. A preparation in the form of a tooth filling, construction element, crown, bridge, part or full prosthesis, abutment or root post (10, 11, 12, 13, 14, 15, 16, 17) to be used in a method for fixing to a natural tooth part or tooth, in particular for replacement of a load-bearing tooth part, in which method in a first step a natural tooth part or tooth is prepared, in a second step the preparation (10, 11, 12, 13, 14, 15, 16, 17) is positioned in the region of the prepared tooth part or tooth in such a manner that the material with thermoplastic properties is in contact or can be brought into contact with a dentin surface and/or an enamel surface of the natural tooth part or tooth, and in a third step the preparation is excited into mechanical oscillation and simultaneously pressed against the tooth part or tooth, wherein for exciting with mechanical oscillation and for pressing, the preparation is pressed against the natural tooth part or tooth with the aid of a sonotrode (50) of an ultrasound apparatus or with the aid of a coupling piece (51) being positioned between the sonotrode (50) and the natural tooth or tooth part and the sonotrode, and wherein in the third step mechanical oscillations are used with a frequency in the region between 2 and 200 kHz, wherein the preparation comprises at least one region (10.2, 12.2, 14.2, 15.2, 16.2, 17.2) or one part (11.2) of a material with thermoplastic properties, wherein this material forms at least a part of the preparation surface or is positionable to form at least a part of the preparation surface, wherein the preparation comprises oscillation properties with damping losses which are so small that local stress concentrations are required for liquefying the material by oscillation, wherein the preparation is designed in such a manner that the stress concentrations only occur in the region of the preparation surface, and wherein the material with thermoplastic properties comprises a modulus of elasticity which is larger than 0.5 Gpa.

2. The preparation (10,11,12,13,14,15,16,17) according to claim 1, **characterized in that** the material with thermoplastic properties is a thermoplastic material or a composite material with a thermoplastic component.

3. The preparation (10, 11, 12, 13, 14, 15, 16, 17) according to claim 2, **characterized in that** the thermoplastic material or the thermoplastic component is a polyolefin, a polyacrylate, a polymetacrylate, a polyurethane, a polycarbonate, a polyamide, a polyester, a polysulphone, a polyarylketone, a polyimide, a polyphenyl sulphide, a liquid crystal polymer, a polyacetal, a halogenated polymer, in particular a halogenated polyolefin, a polyphenylene sulphide, a polysulphone" a polyether or a corresponding copolymer or mixture of at least two of said polymers.

4. The preparation according to one of claims 1 to 3, **characterized in that** the material with thermoplastic properties is a thermoplast with admixtures of fibres, whiskers, particles, agents acting antiseptically or in a denaturising manner, or x-ray absorbing constituents.

5. The preparation according to one of claims I to 3, **characterized in that** the material with thermoplastic properties comprises an active component reacting with the surface of the natural tooth part or with a primer deposited thereon.

6. The preparation (10, 11, 12, 13, 14, 15, 16, 17) according to one of claims 1 to 5, **characterized in that** it comprises a further region (10.1,12.1,14.1.15.1,16.1, 17.1) or part (11.1) of a metallic or ceramic material, of a polymer or of a composite material.

7. The preparation (10, 11, 12, 13) according to one of claims 1 to 6, **characterized in that** it has the shape of a tooth filling to be fixed in a tooth cavity (2), or the shape of an element (41) to be fixed to a tooth (40).

8. The preparation (10, 11, 12) according to claim 6, **characterized in that** it comprises a preparation body (10.1, 11.1, 12.1) and a contact layer (10.2, 12.2) arranged on the preparation body (10.1, 11.1, 12.1), or a contact film (11.2) 1'orming a separate preparation part.

9. The preparation (13) according to claim 6, **characterized in that** it consists completely of the material with thermoplastic properties.

10. The preparation (14) according to one of claims 1 to 6, **characterized in that** it can be fixed upon a natural tooth stump (20), and that the material with the thermoplastic properties forms an inner surface of the crown, abutment, construction clement, bridge or prosthesis or is positionable on this inner surface.

11. The preparation (15, 16) according to one of claims 1 to 6, **characterized in that** it comprises a fastener body (15.1, 16.1) to be fixed in a cavity of a natural tooth part in preparation for the fastening a further, artificial tooth part.

12. The preparation (16) according to claim 11, **characterized in that** it comprises at least one root part (16.2) arranged on the fastener body (16.1), the root part comprising an elastically or plastically deformable core (16.3), and a covering (16.4) of the material with thermoplastic properties arranged around the core (16.3).

13. The preparation (16) according to claim 12, **characterized in that** at the distal end of the core (16.3) there is arranged a thickening (16.5) of the core material or a plug (16.6) of a soft thermoplast.

14. The preparation (17) according to one of claim5 1 to 6, **characterized in that** it comprises a crown part (17.1) and a connection body (17.2), wherein the connection body (17.2) consists at least partly of the material with thermoplastic properties and is designed in such a manner that it is positionable at least partly in a cavity of a natural tooth part.

15. A non-therapeutic method for fastening a preparation, which preparation comprises at least one region (10.2, 12.2, 14.2, 15.2, 16.2, 17.2) or one part (11.2) of a material with thermoplastic properties, wherein this material forms at least a part of the preparation surface or is positionable to form at least a part of the preparation surface, wherein the preparation comprises oscillation properties with damping losses which arc so small that local stress concentrations are required for liquefying the material by oscillation, wherein the preparation is designed in such a manner that the stress concentrations only occur in the region of the preparation surface, on a natural tooth part or tooth, **characterized in that** in a first step a natural tooth part or tooth is prepared, that in a second step the preparation (10, 11, 12, 13, 14, 15, 16, 17) is positioned in the region of the prepared tooth part or tooth in such a manner that the material with thermoplastic properties is in contact or can be brought into contact with a dentin surface and/or an enamel surface of the natural tooth part or tooth, and that in a third step the preparation is excited into mechanical oscillation and simultaneously pressed against the tooth part or tooth, wherein for exciting with mechanical oscillation and for pressing, the preparation is pressed against the natural tooth part or tooth with the aid of a sonotrode (50) of an ultrasound apparatus or with the aid of the sonotrode and a coupling piece (51) being positioned between the sonotrode (50) and the natural tooth or tooth part, wherein in the third step mechanical oscillations are used with a frequency in the region between 2 and 200 kHz, and wherein the material with thermoplastic properties comprises a modulus of elasticity which is larger than 0.5 Gpa.

16. The method according to claim 15, **characterized in that** in the first step dentin and/or enamel surfaces are roughened, are provided with retention structures and/or arc pre-treated with a primer and/or a sealing means.

17. The method according to one of claims 15 or 16, **characterized in that** a two-part preparation (11) is used and that in the second step firstly a first preparation part (11.2) comprising the material with the thermoplastic properties is positioned and made to oscillate, and then a second preparation part (12.1) is positioned and made to oscillate.

## Revendications

1. Préparation sous la forme d'un remplissage dentaire, d'un élément de construction, d'une couronne, d'un bridge, d'une prothèse partielle ou d'une prothèse intégrale, d'un aboutement, ou d'une goupille de racine dentaire (10, 11, 12, 13, 14, 15, 16, 17), destinée à être utilisée dans un procédé et à être fixée sur une partie de dent naturelle ou sur une dent, en particulier pour le remplacement d'une partie de dent qui supporte une charge, dans laquelle, au cours d'une première étape, une partie de dent naturelle ou la dent est préparée, et, au cours d'une seconde étape, la préparation (10, 11, 12, 13, 14, 15, 16, 17) est positionnée dans la zone de la partie de dent préparée ou de la dent préparée de telle sorte que le matériau avec des propriétés thermoplastiques vient en contact ou est amené en contact avec une surface dentine et/ou avec une surface de fusion de la partie de dent naturelle ou de la dent et en ce que, au cours d'une troisième étape, la préparation est excitée sous des oscillations mécaniques et est simultanément pressée contre la partie de dent ou contre la dent et dans laquelle la préparation est pressée contre la partie de dent naturelle ou contre la dent pour l'excitation avec des oscillations mécaniques et pour le pressage avec une sonotrode (50) d'un appareil à ultrasons ou avec la sonotrode (50) et un élément d'accouplement (51) positionné entre la sonotrode (50) et la préparation et dans laquelle, au cours de la troisième étape, des oscillations mécaniques sont utilisées avec une fréquence dans la plage entre 2 et 200 Khi, la préparation présentant au moins une zone (10.2, 12.2, 14.2, 15.2, 16.2, 17.2) ou une partie (11.2) qui est constituée d'une matériau avec des propriétés thermoplastiques, et dans laquelle ce matériau forme au moins une partie de la Surface de la préparation ou est au moins une partie de la surface de la préparation en formant une possibilité de positionnement, et dans laquelle la préparation présente des propriétés d'oscillation avec de telles petites pertes d'amortissement de telle sorte que, pour une liquéfaction du matériau avec des propriétés thermoplastiques au moyen des oscillations, des concentrations locales de contrainte sont nécessaires et dans laquelle la préparation est réalisée de telle sorte que les concentrations de contrainte surgissent seulement dans la zone de la surface, de préparation et dans laquelle le matériau avec des propriétés thermoplastiques présente un module d'élasticité qui est supérieur à 0,5 GPa.

2. Préparation (10, 11, 12, 13, 14, 15, 16, 17) selon la revendication 1, **caractérisée en ce que** le matériau avec des propriétés thermoplastiques est un matériau thermoplastique ou un matériau mixte avec un composant thermoplastique.

3. Préparation (10, 11, 12, 13, 14, 15, 16, 17) selon la revendication 2, **caractérisée en ce que** le matériau thermoplastique ou le composant thermoplastique est une polyoléfine, un polyacrylate, un polyméthacrylate de méthyle, un polyuréthane, un polycarbonate, une polyamide, un polyester, un polysulfone, une acétone polyarylique, une polyimide, un sulfure polyphénylique, un polymère cristallin liquide, un polyacétal, un polymère halogénisé, en particulier une polyoléfine halogénisée, un sulfure polyphénylique, un polysufone ou un polyester ou un copolymère correspondant ou un mélange d'au moins deux des polymères mentionnés.

4. Préparation selon l'une des revendications 1 à 3, **caractérisée en ce que** le matériau avec des propriétés thermoplastiques est un thermoplaste avec des mélanges de fibres, de cristaux fins, de particules, d'agents à action antiseptique ou dénaturée ou de composants qui absorbent les rayons X.

5. Préparation selon l'une des revendications 1 à 3, **caractérisée en ce que** le matériau avec des propriétés thermoplastiques présente des composants actifs qui réagissent avec une surface de la partie de dent naturelle ou avec un apprêt appliqué dessus.

6. préparation (10, 11, 12, 13, 14, 15, 16, 17) selon l'une des revendications 1 à 5, **caractérisée en ce qu'**elle présente une autre zone (10.1, 12.1, 14.1, 15.1, 16.1, 17.1) ou une partie (11.1) qui est constituée d'un matériau métallique ou céramique, d'un polymère ou d'un matériau mixte ou d'un matériau composite.

7. Préparation (1 D, 11, 12. 13) selon l'une des revendications 1 à 6, **caractérisée en ce qu'**elle a la forme d'un remplissage dentaire qui doit être mis en place dans une cavité de dent (2) ou d'un élément (41) qui doit être fixé sur une dent (40).

8. Préparation (10, 11, 12) selon la revendication 6, **caractérisée en ce qu'**elle présente un corps de préparation (10.1, 11.1, 12.1) et une couche de contact (10.2, 12.2) qui est agencée sur le corps de préparation (10.1, 11.1, 12.1) ou un film de contact (11.2) qui l'orme une partie séparée de la préparation.

9. Préparation (13) selon la revendication 6, **caractérisée en ce qu**'elle est constituée complètement du matériau avec des propriétés thermoplastiques.

10. Préparation (14) selon l'une des revendications 1 à 6, **caractérisée en ce qu'**elle peut être mise en place sur un chicot naturel de dent (20) et **en ce que** le matériau avec des propriétés thermoplastiques forme une surface intérieure de la couronne, de l'aboutement, de l'élément de construction, du bridge ou de la prothèse ou peut être positionné dessus.

11. Préparation (15, 16) selon l'une des revendications 1 à 6, **caractérisée en ce qu'**elle présente un corps de fixation (15.1, 16.1) qui peut être mis en place dans une cavité d'une partie de dent naturelle pour la fixation d'une autre partie de dent artificielle.

12. Préparation (16) selon la revendication 11, **caractérisée en ce qu'**elle présente au moins une partie de racine (16.2) agencée sur le corps de fixation (16.1) avec un noyau (16.3) qui est déformable élastiquement ou plastiquement et une enveloppe (16.4) agencée autour du noyau (16.3) qui est constituée du matériau avec des propriétés thermoplastiques.

13. Préparation (16) selon la revendication 12, **caractérisée en ce qu'**une boursouflure (16.5) depuis le matériau de noyau ou un bouchon (16.6) est constituée d'un thermoplaste mou et est agencée sur l'extrémité distale du noyau (16.3).

14. Préparation (17) selon l'une des revendications 1 à 6, **caractérisée en ce qu'**elle présente une partie de couronne (17.1) et un corps de liaison (17.2), dans laquelle le corps de liaison (17.2) est constitué au moins en partie du matériau avec des propriétés thermoplastiques et est réalisé de telle sorte qu'il puisse être positionné au moins en partie dans une cavité d'une partie de dent naturelle.

15. Procédé non thérapeutique pour la fixation d'une préparation (10, 11, 12, 13, 14, 15, 16, 17), la préparation présentant au moins une zone (10.2, 12.2, 14.2, 15.2, 16.2, 17.2) ou une partie (11.2) qui est constituée d'une matériau avec des propriétés thermoplastiques, et dans laquelle ce matériau forme au moins une partie de la surface de la préparation ou est au moins une partie de la surface de la préparation en formant une possibilité de positionnement, et la préparation présentant des propriétés d'oscillation avec de telles petites pertes d'amortissement de telle sorte que, pour une liquéfaction du matériau avec des propriétés thermoplastiques au moyen des oscillations, des concentrations locales de contrainte sont nécessaires et dans laquelle la préparation est réalisée de telle sorte due les concentrations de contrainte surgissent seulement dans la zone de la surface de préparation sur une partie de dent naturelle ou sur une dent, **caractérisé en ce que**, au cours d'une première étape, une partie de dent naturelle ou la dent est préparée, et **en ce qu'**au cours d'une seconde étape, la préparation (10, 11, 12, 13, 14, 15, 16, 17) est positionnée dans la zone de la dent ou de la partie de dent préparée de telle sorte que le matériau avec des propriétés thermoplastiques vient en contact ou peut être amené en contact avec une surface dentine et/ou avec une surface de fusion de la partie de dent naturelle ou de la dent, et **en ce que**, au cours d'une troisième étape, la préparation est excitée à des oscillations mécaniques et est simultanément pressée contre la partie de dent ou contre la dent, et dans laquelle la préparation est pressée contre la partie de dent naturelle ou contre la dent pour l'excitation avec des oscillations mécaniques et pour le pressage avec une sonotrode (50) d'un appareil à ultrasons ou avec un élément d'accouplement (51) positionné entre la sonotrode (51) et la préparation et dans laquelle, au cours de la troisième étape, des oscillations mécaniques sont utilisées avec une fréquence dans la plage entre 2 et 200 KHz et dans laquelle le matériau avec des propriétés thermoplastiques présente un module d'élasticité qui est supérieur à 0,5 GPa.

16. Procédé selon la revendication 15, **caractérisé en ce que**, au cours d'une première étape, la surface dentine et/ou la surface de fusion sont rendues rugueuses, sont munies de structures de maintien ct/ou d'un apprêt et/ou sont prétraitées avec un moyen de scellement.

17. Procédé selon l'une ou l'autre des revendications 15 et 16, **caractérisé en ce que** l'on utilise une préparation (11) en deux parties et **en ce que**, au cours d'une seconde étape, une première partie de préparation (11.2) qui présente des propriétés du matériau avec des propriétés thermoplastiques, est tout d'abord positionnée et est sollicitée avec les oscillations et **en ce qu'**ensuite, une seconde partie de préparation (12.1) est positionnée et est sollicitée avec les oscillations.
